# EUROPEAN PATENT APPLICATION

(11) **EP 4 108 196 A1**
(43) Date of publication of application: **28.12.2022**
(21) Application number: 21181602.0
(22) Date of filing: 24.06.2021
(51) Int. Cl.: A61B 18/14

(54) **DEVICE FOR REMOVAL OF MICROBIAL CONTAMINANTS**

(71) Applicant: National University of Ireland Galway, Galway (IE)
(72) Inventor: Markey, Lyn, Dublin, D18H265 (IE); O'Malley, Camille, Galway (IE); DeCaro, Cristoforo, Galway (IE); O'Halloran, Martin, Galway (IE)
(74) Representative: FRKelly

(57) **Abstract**

The present disclosure relates to a device and methods for removing microbial contaminants from a tissue environment. The device comprises a non-invasive distal portion including a plurality of electrodes configured to provide a pulsed electric field (PEF) signal on a tissue surface in the vicinity of a microbial contaminant, wherein the PEF penetrates beneath the tissue surface.

## Description

### TECHNICAL FIELD

The present invention relates to a device and method for removal of microbial contaminants. In particular, the invention has application in the removal of a microbial contaminants in the presence of mammalian tissue.

### BACKGROUND

Microbial contaminants can exist as single, free floating cells (planktonic) or in densely packed communities called a biofilm. Biofilms have a chemical and physical tolerance to anti-microbials and host immunity. Between 65 - 80% of infections can be attributed to biofilms including diabetic foot infections (including those that progress to osteomyelitis), prosthetic joint infections (PJI), osteomyelitis, chronic wounds, chronic sinusitis, burns, surgical wounds, traumatic wounds, acne, etc. Microbial cells within a biofilm grow at a slower pace than planktonic microbes as there are limited resources and therefore are poor targets for antibiotics that target growth inhibition. More importantly, microbes within a biofilm are surrounded by an extracellular matrix (ECM) made up of proteins, nucleic acids, and polysaccharides. This acts as a physical barrier against both host immunity and antibiotics.

Currently, treatments have a two-step approach - physically remove microbial contaminants through irrigation and debridement procedures and treat residual microbes with suitable antibiotics. The clinical gold standard of treatment for moderate to severe infections is operative debridement of the necrotic tissue and physical removal of microbial contaminants from the site. However, current treatments of these patients are wholly ineffective; a recent study found that typically patients require two or more surgeries for infection control.

Debridement procedures typically use physical (scalpel and brush) and chemical (flushed saline, antiseptic solutions) to remove microbial contaminants from the wound and devitalized tissue. Newer innovative devices use pressure to physically disrupt the microbial contaminants. Increased pressure within the wound can initially remove more microbial contaminants however there is evidence to suggest it may distribute microbes further down into tissue as there is a higher rebound of bacteria several hours after surgery. Moreover, stronger antiseptics can marginally reduce bacterial burden, compared to saline, but also cause inflammation in host tissue and impact wound healing. Large doses of antibiotics placed in the wound can prevent wound healing and cannot diffuse to all areas within the infected space.

The costs associated with treating diabetic foot ulcers (DFU) is between US$ 9 - 13Bn in the United States. A very small percentage of DFU's account for the majority of healthcare costs. It is estimated that 74 - 84% of costs are utilized in inpatient care, accounting for only 9-20% of DFU patients. Costs are driven by length of stay in hospital, intensive care, and surgical interventions. In 2010, a study showed the average costs of treating a superficial ulcer was as low as $3096 compared to $107,900 for an ulcer resulting in amputation.

When microbial contaminants are present as a biofilm, microbes are up to 1000 times more tolerant to antimicrobials than planktonic bacteria. Current treatments do not ensure full coverage of the infected wound or surgical site - both surface and sub-surface. Furthermore, as there is not full eradication of released microbes, there is a risk of migration of infection to virgin sites.

Presently, there are no approved medical devices that effectively disrupt and eradicate microbial contaminants present as a biofilm. There is therefore a need for a solution which addresses at least some of the deficiencies highlighted above.

### SUMMARY

In a first aspect of the invention, there is provided a device for removing microbial contaminants, comprising a non-invasive distal portion including a plurality of electrodes configured to provide a pulsed electric field, PEF, signal on a tissue surface in the vicinity of a microbial contaminant, wherein the PEF penetrates beneath the tissue surface.

Optionally, the tissue engaging surfaces are axially moveable relative to a horizontal plane and/or relative to a vertical plane. This may allow the device head to move more easily across an irregular topography on the surface of the tissue environment.

Optionally, the device further comprises an insulating material at least partially surrounding a perimeter of at least one electrode for directing the PEF beneath the surface of the tissue. In addition or alternatively, the electrodes comprise a coating which provides resistance to the adhesion of matter.

Optionally, the electrodes are embedded in an insulating material for directing the PEF beneath the surface of the tissue. In addition or alternatively, the electrodes comprise a coating which provides resistance to the adhesion of matter.

Optionally, the device may further comprise at least one impedance sensor to assist in determination of PEF parameters to deliver desired current delivery. As will be discussed ahead in greater detail, different types of tissue exhibit different impedance values, and thus will require different PEF parameters to ensure constant current delivery to the treatment area.

Optionally, the device may further comprise a controller being configured to change one or more parameters of the PEF signal in response to the at least one impedance sensor detecting an impedance value or a change in impedance.

Optionally, the controller is configured to determine electrical properties of tissue based on an impedance value detected by the at least one impedance sensor, and/or the controller being configured to change one or more parameters of the PEF signal in response to determining electrical properties of tissue.

Optionally, the device further comprises suction means for providing suction in the vicinity of the microbial contaminants. This may facilitate the removal of debris from the vicinity of the tissue environment before, during, and/or after treatment.

Optionally, the distal portion may further comprise a volume configured for providing an at least partially sealed environment when in abutment with a surface. The provision of suction means such as that described above may also augment the seal created in embodiments where the device comprises the said volume.

Optionally, the volume is at least partially formed of a resilient material. This may allow for improved surface contact between the tissue environment and the distal portion of the device, further augmenting the seal created. A resilient material will return to its former shape once an applied force is removed from it, thus allowing the volume to return to its rest shape after use. Further, in embodiments where the electrodes neither sit proud of the device head nor are flush with the end of the device head, it may be useful for the volume to be resilient such that under pressure the electrodes may be moved in to contact with the tissue surface.

Optionally, the tissue engaging surfaces are protruding and/or flat relative to the device head and/or comprise a smooth or irregular surface.

Optionally, the device further comprises a dispensing means configured to dispense a fluid to the vicinity of the microbial contaminants. The fluid may be a conductive fluid, and/or an antiseptic fluid, such as a saline fluid. The provision of a conductive fluid may enhance the distribution of current around the treatment area, whilst an antiseptic fluid will help in removing further potential infections to the wound bed.

Optionally, the distal portion of the device head may be at least partially formed of an adaptable/flexible material to conform to irregular topographies. This will help to enhance the surface area contact between the distal portion and the surface of the tissue environment.

Optionally, the distal portion of the device may contain sensors to detect the physical environment including combinations of temperature, pressure, pH, and the position of electrodes.

Optionally, the device may further comprise a proximal portion on which the non-invasive distal portion is mounted.

According to a second aspect of the invention, there is provided a method of removing microbial contaminants, comprising providing a pulsed electric field (PEF) signal to the vicinity of the microbial contaminants.

According to a third aspect of the invention there is provided a method of directing a PEF signal to tissue to regenerate the wound bed and aid in the wound healing process by stimulating growth factors and promoting angiogenesis, comprising providing a pulsed electric field (PEF) signal to the vicinity of the wound.

Optionally, the methods further comprise:
detecting, by an impedance sensor, an impedance value or a change in impedance; and
changing one or more parameters of the PEF signal in response to detecting the impedance value or the change in impedance.

Optionally, the PEF signal provides a current between at least two adjacent electrodes. In addition or alternatively, the methods further comprise selectively reversing the polarity of a pair of electrodes. The polarity change in the system allows for more effective disruption of cellular components in the tissue.

Optionally, the methods further comprise the steps of:
providing a volume to facilitate an at least partially sealed environment; and providing suction.

Optionally, the methods further comprise providing a conductive fluid to the vicinity of the microbial contaminants; or providing an antiseptic fluid to the vicinity of the microbial contaminant.

Optionally, the PEF signal is provided on the tissue surface, and the PEF penetrates beneath the tissue surface.

Optionally, the method further comprises providing a PEF signal to create pores in cell membranes and enhance delivery of therapies to cells.

Optionally, the method further comprises providing a PEF signal to enhance wound regeneration by increased growth factor and/or angiogenesis.

### BRIEF DESCRIPTION OF THE DRAWINGS

Embodiments of the invention shall now be described with reference to the following figures which are provided by way of example:
Figures 1-3 provide illustrations of a device for use in removing microbial contaminants, according to embodiments of the present disclosure.
Figure 4 provides a method of removing microbial contaminants according to an embodiment of the present disclosure.
Figure 5 provides a block diagram of an exemplary feedback system for adjusting the electric field strength of a pulsed electric field according to an embodiment of the present disclosure.
Figure 6 provides a schematic diagram illustrating a setup in which a human-controlled console is used to control, via actuators, the exemplary system;
Figure 7 provides views of the device head according to two different embodiments having different numbers of electrodes;
Figure 8 provides illustrations of a plurality of different possible electrode configurations of the exemplary system;

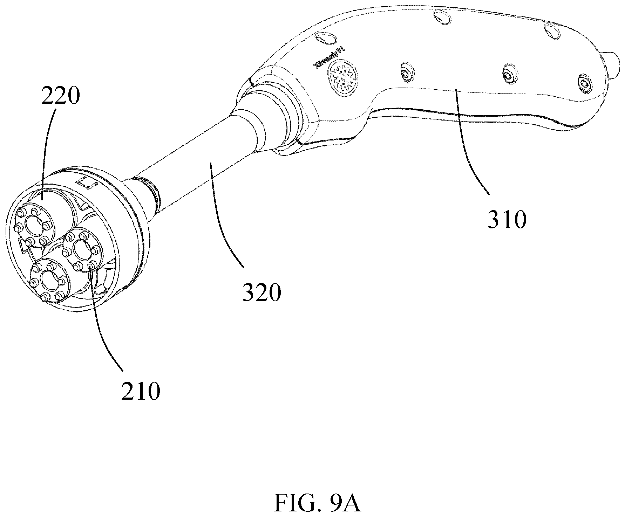

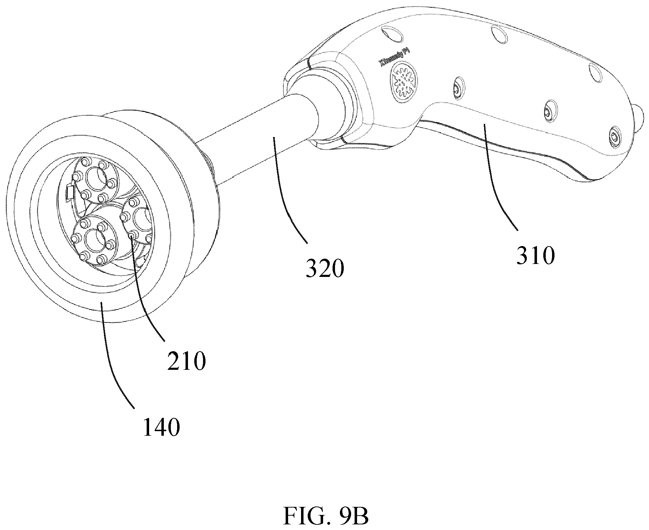

Figures 9A and 9B provide views of the exemplary system including the proximal portion, and optionally including a volume on the device head;

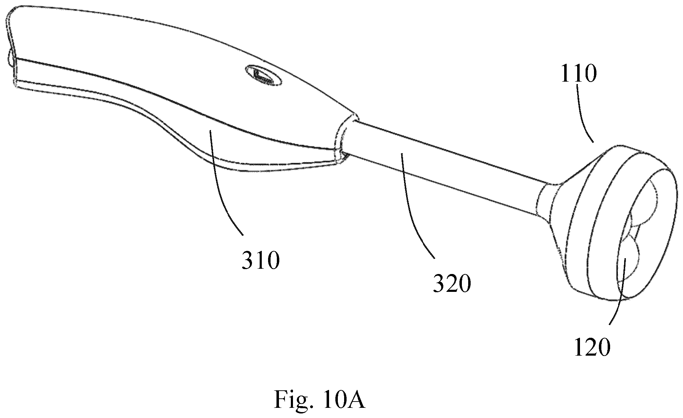

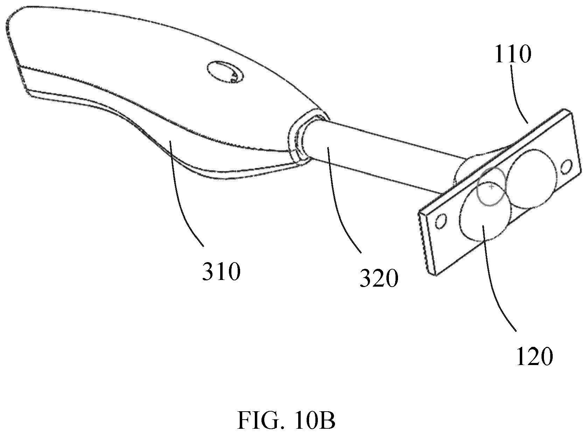

Figures 10A and 10B provide further views of the exemplary system including the proximal portion, according to embodiments of the present disclosure; and
Figure 11 provides a conceptual illustration of electrodes embedded in an insulating material, according to embodiments of the present disclosure.

### DETAILED DESCRIPTION

The present teaching will now be described with reference to an exemplary device and method for removal of microbial contaminants, and in particular for removal of microbial contaminants. It will be understood that the exemplary method and system is provided to assist in an understanding of the present teaching and are not to be construed as limiting in any fashion. For example, whilst the disclosure focuses primarily on the removal of biofilm, the systems and methods described herein may be used in the removal of other potential microbial contaminants. The term "tissue environment" is used in several places with reference to the treatment area, which can include one or more types of tissue. Furthermore, elements or components that are described with reference to any one Figure may be interchanged with those of other Figures or other equivalent elements without departing from the spirit of the present teaching.

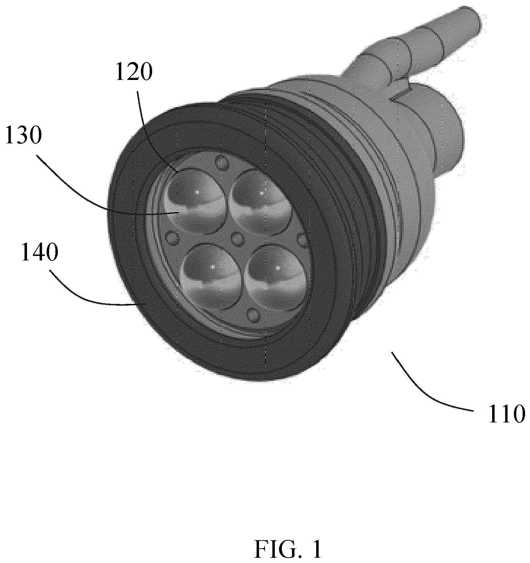

Referring to Figure 1, there is illustrated a device for removal of microbial contaminants (not visible), comprising a distal portion 110. The distal portion 110 includes a plurality of electrodes 120 configured to provide an electromagnetic signal in the vicinity of the microbial contaminants to facilitate electroporation of both microbial contaminants and surrounding tissues. Preferably, the electromagnetic field provided by the electrodes 120 is a pulsed electric field (PEF). Current therapies fail to disrupt microbial contaminants or the structural integrity of the extracellular matrix (ECM) of the biofilm and thereby antiseptic solutions cannot interact with protected microbes inside the film. PEFs induce a transmembrane potential on microbial cells and at a threshold, the potential can induce permeabilization of the cell membrane through creation of pores. The intensity of the transmembrane potential will determine if the permeability is reversible or irreversible. Inactivation of biofilm ECM is a result of this permeabilization and/or the production of reactive oxygen species. PEF's can further cause microbial cell wall morphological changes and degradation, and thereby enable its removal. PEF also creates an electrical field across cell membranes that can produce irreversible pores in their membrane leading to cell death. Given that microbial cells are typically smaller than mammalian cells, they can be targeted with minimal damage to host tissue. Transmembrane potentials are proportional to the organism size therefore higher field strengths are required for bacteria which limits any damage to host tissue. Macrostructures, such as nerve cells and blood vessels, are not damaged by PEF. In addition, the electrical field generated by the device may stimulate growth and regeneration of a wound bed.

Each electrode defines a tissue engaging surface 130. In some embodiments the tissue engaging surfaces are conformal to the tissue surface and/or non-invasively transmit signal below the surface of the tissue when in an engagement state. It will be understood that whilst the electrodes define "tissue engaging surfaces", this does not preclude that a medium, such as a gel, may be located on the surface of the tissue itself, and in-use the tissue engaging surfaces may contact the tissue and/or the medium. Accordingly, the terms "non-engagement state" and "engagement state" should not be construed as being limited to embodiments in which the electrodes are in direct contact with the tissue surface since a medium may be located therebetween. Previously, the electrode configuration for non-invasive treatment of bacteria in a clamp type, 180° vis-a-vis configuration, which pinches the tissue/skin in order to minimise the distance between the electrodes and direct current through the tissue. This can only be applied to treat superficial wounds where skin is malleable to position between electrodes. In some embodiments, the tissue engaging surfaces 130 may be axially moveable relative to a horizontal plane and, optionally, the distance between the longitudinal axes of an adjacent pair of electrodes 120 may be maintained and/or determined. For example, when the distal portion 110 is moved in to contact with tissue, the tissue engaging surfaces 130 may be axially moveable relative to the tissue surface whilst the axes up and down which the tissue engaging surfaces 130 can move. Configuring the tissue engaging surfaces 130 to move axially in this way aids the exemplary device in moving over irregular surfaces whilst in the engagement state. In some embodiments, the tissue engaging surfaces may be axially moveable relative to the vertical plane. This further improves the usability of device for the user and its efficacy by augmenting surface area contact between the tissue engaging surfaces 130 and the tissue. The tissue engaging surfaces 130 may be configured in a number of different ways to be axially movable. In various embodiments, the electrodes 120 may each be resiliently biased. In one embodiment, the electrodes 120 may each be resilient biased by means of a mechanism to engage contact.

In the embodiment of Fig. 1, the electrodes 120 comprise a plurality of discs arranged concentrically about the distal portion 110. In the exemplary embodiment, the electrodes 120 are equally spaced apart. By way of example only, the electrodes may be spaced apart from one another by 0.2cm. The tissue engaging surfaces 130 may each define a curved geometry to enhance surface contact. This rounded electrode shape allows for full surface area contact with tissue, reducing the natural impedance of the human body and allowing more efficient signal delivery. In some embodiments, the tissue engaging surfaces may comprise indentations (not presented) to further enhance surface area and allow the current to be transferred deeper into the tissue. In some embodiments, the electrode platform is/are malleable to be conformed to a desired shape, such as the shape of one or more wound areas intended for treatment. In certain embodiments, the electrodes 120 may comprise a coating which provides resistance to the adhesion of matter, i.e. a "non-stick" coating, to prevent buildup of layers caused by oxidation, debris, and such, on the electrodes which impacts the ability of the electrodes 120 to deliver the correct amount of current to the area. In some embodiments the electrodes are coated to enhance signal delivery. The coating may be located on the tissue engaging surfaces 130. In some embodiments, the electrodes 120 are designed with smooth edges to reduce damage to tissue and prevent the edge effect.

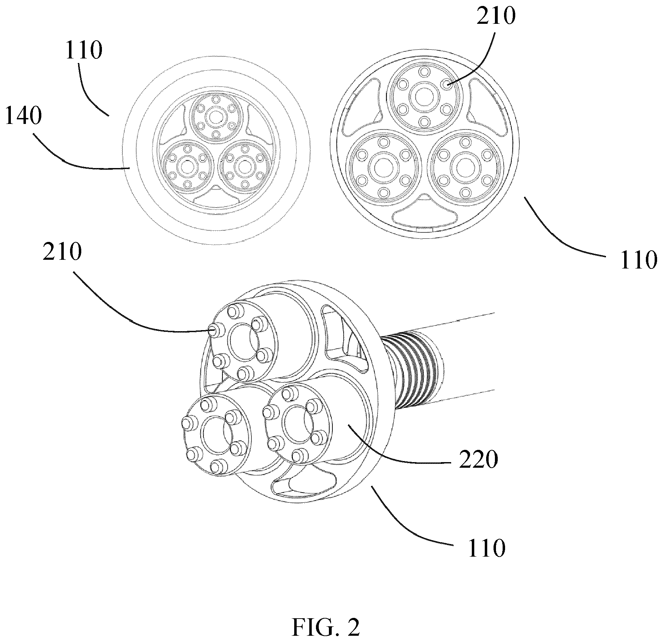

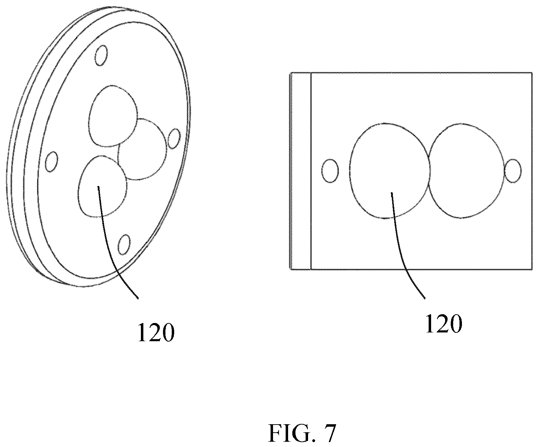

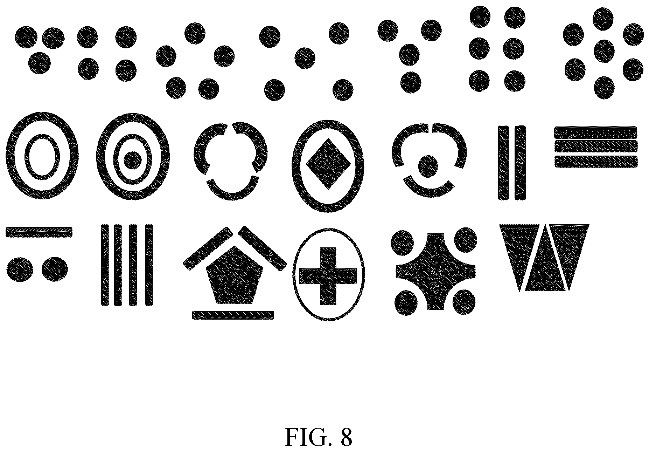

Whilst four electrodes 120 are depicted in Figure 1, other numbers are envisaged. For example, it may be possible to use one, two, three or four electrodes 120, and so on. Alternative embodiments of the electrodes are envisaged as being feasible, such as but not limited to a plurality of electrode pins 210 mounted on electrode pin holders 220 on the distal portion 110, Figure 2. In addition, whilst discs 120 are presented in Figure 1, other shapes of electrodes 120 may also be feasible including but not limited to circular, oval, rectangular, square and irregular shapes. The electrodes 120 may be formed from a number of appropriate materials, such as but not limited to copper, gold, platinum, iridium, stainless steel, silver, alloys, carbon or another conductive material. The electrodes are depicted in Figure 1 as having a rounded or protruding shape, allowing the electrodes to sit proud of the device head. In some embodiments the electrodes can be in-line with the device head. In other embodiments the electrodes may sit within the device head in a conformation to direct signal to the tissue. The exemplary embodiment of Figure 7 shows the electrodes 120, in a configuration of 2 and 3 electrode patterns. In some embodiments the configuration of the tissue engaging surfaces are circular, concentrical, linear, oblique and/or crown configurations. The exemplary configurations (Figure 8) can be achieved with multiple electrode shapes including but not limited to circular, oval, rectangular, square and irregular shapes.

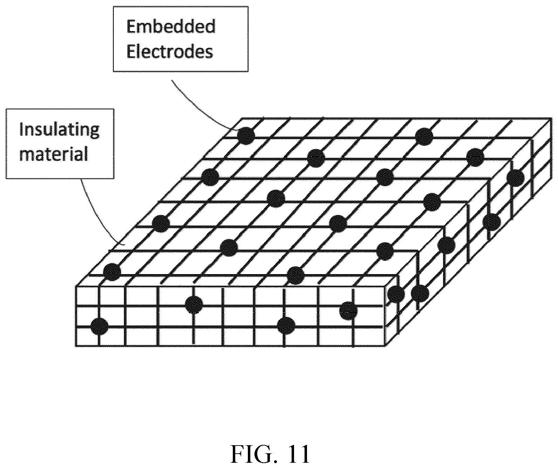

In the exemplary embodiment, the electrodes 120 comprise an insulating material at least partially surrounding a perimeter of each electrode 120. In some embodiments, the electrodes 120 are embedded in an insulating material, best presented in Figure 11. These configurations using the insulating material may help to prevent current density pooling between electrodes 120, such that current flows between at least two adjacent tissue engaging surfaces 130. The insulating material therefore augments the ability of the exemplary device to direct current to the treatment area.

Also visible in Figure 1 is a volume 140 located at one end of the distal portion 110. The volume 140 can mitigate the spread of debris, microbes, and the like from the treatment area by providing an at least partially sealed environment when in abutment with a surface. In some embodiments, the volume 140 is at least partially formed of a resilient material, such that when pressure is applied to the surface the volume 140 bends slightly under pressure. The volume 140 may be formed of a variety of suitable materials, such as but not limited to silicone or various polymers comprising the appropriate mechanical properties.

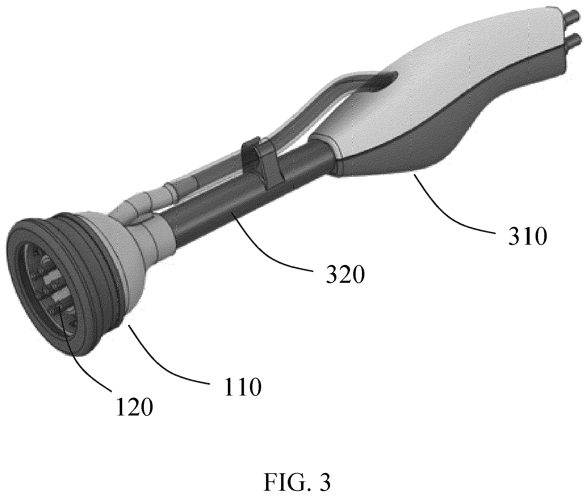

Once treated, biofilm matrix components can breakdown to release planktonic bacteria within a wound. Accordingly, the device may also comprise suctions means (not visible) which, when used in conjunction with the volume 140, is particularly effective in removing of debris, microbes, and the like from the treatment area. To facilitate suction means, at least one opening may be provided at the distal portion 110. Then, for example, an out-port, best presented in Figure 3, may be provided leading from said opening away from the distal portion 110. The suction means may be suitably pressurised to a desired pressure to provide suction and facilitate passage of debris, microbes, and the like. For example, an external aspirator means or vacuum means may be connected to out-port to provide the required pressurisation. In addition, the suction means may also work to maintain a steady purchase of the surgical site for the duration of a pulse delivery. Under light vacuum, the electrodes 120 are less prone to slip or move from their site during use.

In an advantageous embodiment, the device further comprises dispensing means configured to dispense a fluid to the vicinity of the microbial contaminants. For example, the fluid may comprise a conductive medium, and/or an antiseptic fluid, such as a saline fluid. The use of a conductive fluid together with the PEF may further optimise the delivery and penetration of the electric current to each area of the wound bed; saline or other conductive mediums can propagate the PEF signal to cover a larger surface area and/or enhance signal delivery to an area. Optionally, the volume 140 may help to contain the conductive medium and to limit spread of treated microbes/debris to virgin sites. The dispensing means may comprise an aperture and a conduit for directing the fluid to the aperture and on to the treated area. In some embodiments, the fluid may flow at low pressure into the device head to contact the tissue and the electrodes 120. Where a dispensing means is included in the device, separate in- and out- ports are provided for delivery of the fluid and for suction respectively. In some embodiments, the conductive fluid is delivered in parallel with signal delivery. In some embodiments, a conductive gel is used with or without the volume 140 described above to enhance signal delivery to the area. In some embodiments, the delivery of conductive fluid and/or the operation of pressurising system is controlled by an automatic system. In some embodiments, there is a pump as part of the system to regulate flow of a conductive medium, such as a conductive fluid, to the treatment area. In some embodiments, there is a motor/battery connected to the pump to regulate flow rate. The motor/battery may be external to the proximal part of the device.

Different types of tissue have different values of impedance and, as such, certain parameters of the PEF signal may need to be adjusted during use of the device to ensure a desired current is delivered to the microbial contaminants. The device may therefore comprise one or more impedance sensors to assist in determination of current delivery. For example, the impedance sensor(s) may comprise electrode(s), such as the electrodes 120 visible in the Figures. The device may include a controller configured to change one or more parameters of the PEF signal in response to the impedance sensor(s) detecting an impedance value or a change in impedance. The device, or the system it is part of, may comprise memory storing predefined impedance values and/or predefined threshold changes in impedance which the controller may compare with the measured value or change. The memory may also store sets of PEF parameters set to correspond to certain impedance values; the controller can then alter one or more PEF parameters appropriately based on the particular impedance value detected to optimise treatment. PEF parameters include values of electric field current, voltage, frequency, pulse type (i.e., the shape of the waveform), number of pulses, pulse interval, and pulse duration. In some embodiments, the pulse duration is of the order of microseconds. In some embodiments, the pulse duration is of the order of nanoseconds. In some embodiments, the pulse interval is of the order of seconds. In some embodiments, the pulse interval is of the order of milliseconds. In some embodiments, the pulse type may include a square waveform and/or an exponential waveform. In some embodiments the polarity of electrodes are changed between pulses.

Certain impedance values may correspond to certain types of tissue, such as soft tissue or bone tissue. The controller may therefore be further configured to determine the impedance of tissue based on an impedance value detected by the impedance sensor(s). The controller may be configured to change one or more parameters of the PEF signal in response to determining the impedance of the tissue environment from the impedance sensor(s). The memory may therefore be pre-programmed with PEF parameters for current delivery based on natural tissue impedance and topography. For example, memory may be pre-programmed with a set of PEF parameters corresponding to impedance values associated with tissue type A which becomes relevant when it is determined that the device is in an engagement state with tissue type A.

Figure 3 provides an illustration of the device of the previous Figures mounted on a proximal portion 310. The proximal portion 310 may serve as a handle for the user and/or an attachment means to the tissue and, optionally, may house the in-and/or out- ports for fluid delivery and suction respectively. The proximal portion 310 may also house electrical wiring for providing power to the electrodes 120. In some embodiments, the proximal portion 310 may comprise means to actuate the dispensing means and/or provision of the PEF. In some embodiments, the out-port may be assembled on the top of the device. In some embodiments the proximal portion 310 has an angled handle to aid handling of the device. In some embodiments the proximal portion 310 has an attachment portion for uniting the device to the tissue in a hands-free manner, for example a strap portion may be used to maintain a secure contact of the distal head to the tissue.

Optionally, the proximal portion 310 may at least partially house a multi-lumen shaft 320. The multi-lumen shaft 320 may itself house electrical wires and fluid delivery conduits.

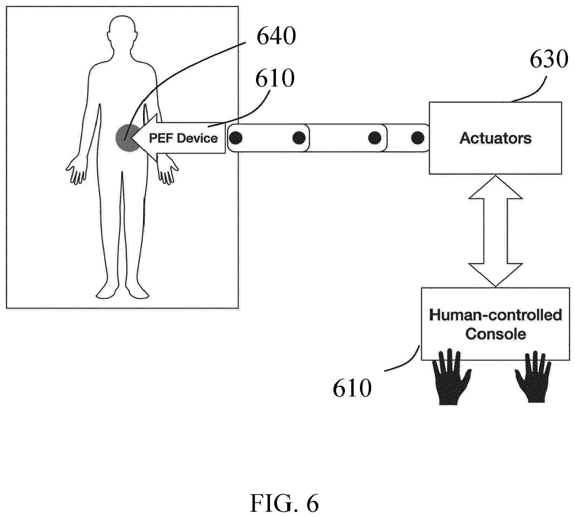

The distal portion 110 may be movably mounted on the proximal portion 310 or the multi-lumen shaft 320. In one embodiment, a ball and socket -type joint may be used, allowing flexible movement in a circular motion of the distal portion 110. This would further improve ease of movement of the device across irregular surfaces during use. In some embodiments, the device is integrated into a robotic system for providing robot-assisted operation, best presented in Figure 6 which provides an illustration of the device 610 controlled remotely through a human controlled console 620. The robotic system includes a set of one or more actuators 630 configured to control the movement and/or the position of the device 620, the robotic-assisted movement provides additional control to fit the area 640 in which the procedure is performed.

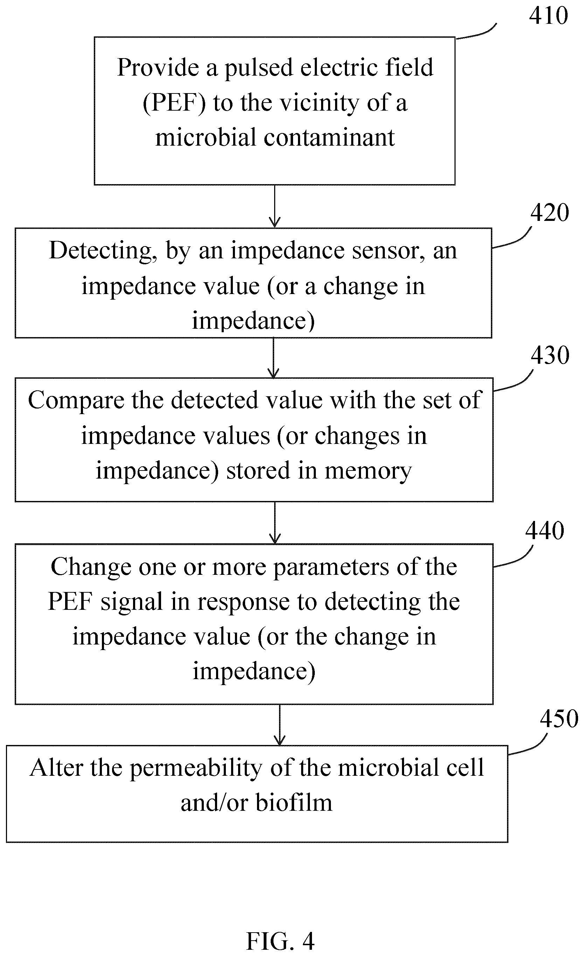

Figure 4 provides an illustration of an exemplary method for removing a microbial contaminant such as a biofilm. The method includes the step 410 of providing a PEF signal to the vicinity of a microbial contaminant. The method optionally includes the step 420 of detecting, by an impedance sensor, an impedance value or a change in impedance. When an impedance value (or change in impedance) is detected by the impedance sensor, the controller may compare the detected value with the set of impedance values (or changes in impedance values) stored in memory, 430. Where the detected impedance value (or detected change in impedance value) does not correspond exactly to a value in the pre-programmed list of impedance values (or changes in impedance values), the controller may make a closest approximation. The controller will match the closest predefined impedance value (or closest change in impedance value) with its corresponding predefined set of PEF parameters, and change one or more parameters of the PEF signal in response to detecting the impedance value (or change in impedance value), 440. The result of providing the PEF in the vicinity of the microbial contaminant is to alter the permeability of the microbial cell and/or biofilm, 450. In some embodiments, pre-set programs will combine user input (type of microbe present, and/or size, shape, site etc. of wound) and detection sensors (impedance, pressure, temperature, pH) to define the optimal program. In some embodiments input data from sensors (such as impedance, pressure, temperature, pH) assist a robotic system for the positioning of the device. Figure 6 describes the remotely human-controlled robotic system, the positioning of the device is assisted by real-time data from sensors that are in-place on the system. In some embodiments, different impedance sensors of the device may detect different impedance values, either at different positions on a same tissue type or at different positions on different tissue type(s). Accordingly, different PEF parameters may be selected as above for different electrodes of the device head. For example, a first impedance sensor A corresponding to a first electrode A may detect a first impedance value Z_{A}, and a second impedance sensor B corresponding to a second electrode B may detect a second impedance value Z_{B}. In response, first and second sets of PEF parameters corresponding to first and second impedance values Z_{A} and Z_{B} may be used for the first and second electrodes, respectively, to ensure constant current delivery for those types of tissues and/or at those positions in the tissue environment.

In some embodiments, the device may be configured with a "fire and forget" auto feedback loop where the treatment is continued until a certain threshold change in impedance is detected by the impedance sensor(s) and/or delivery of PEF parameters to tissue. This ensures full delivery of current to the tissue environment for significant effect of treatment. The reduction in impedance following delivery of high voltage pulses may be measured and can feedback to a user that the treatment of a certain area of tissue/bone is complete.

In alternative embodiments a visual aid on the interface is displayed to demonstrate the output program has been delivered and/or a change in impedance has been detected.

The impedance sensor design and controller automatically feeds the pulse parameter setting on the pulse generator (based on distance between electrodes) to deliver a constant current signal and consistent electric field distribution in the tissue surrounding the electrodes 120. In some embodiments, impedance sensors evaluate the impedance after each current signal delivered and automatically adapts the pulse parameters setting on the generator to deliver a constant current signal.

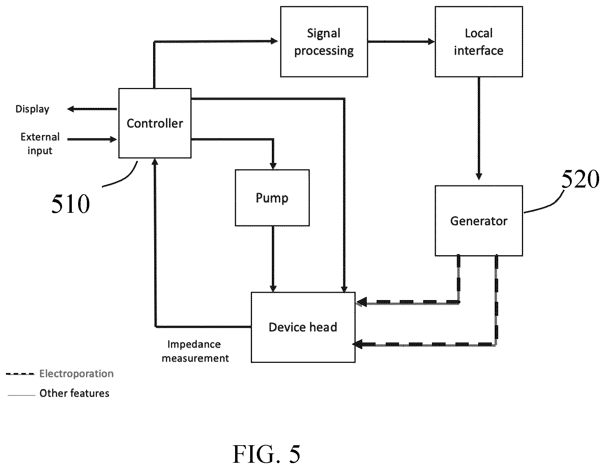

As noted above, different types of tissue have different values of impedance and, as such, certain parameters of the PEF signal may need to be adjusted during use of the device to ensure a desired current is delivered to the microbial contaminants. In particular, it may be desirable that a constant current is delivered at any given time. Referring to Figure 5, there is provided a block diagram of an exemplary feedback system 510 for adjusting the electric field strength of the PEF. Broadband impedance measurements (in Ohms) may automatically interface with the PEF generator 520 to adjust the electric field strength delivered to site. This allows the exemplary device to maintain a constant current system - delivering adequate electric field strength across the treatment region of interest.

Advantageously, the interface system may be used to assist with delivery, positioning, and usability of the device. The system measures impedance measurements from the distal portion 110 to accept/reject electrode 120 positioning for use.

It will be understood that the approximation made by the controller or other components of the device or system may be performed a number of ways. For example, by a simple predefined plus-minus value within which the detected value might fall, or by other more sophisticated methods. In some embodiments, the apparatus employs artificial intelligence (AI) to facilitate delivery of the optimal signal. In some embodiments, the device involves a computer implemented AI algorithm for monitoring live feedback of the treatment and/or optimize the parameters changing the setting. The AI algorithm identifies the optimal setting based on real-time data such as but not limited to impedance, temperature, pressure, distance between electrodes

In one embodiment, the method may further comprise the step of switching the polarity of pairs of electrodes 120. The polarity change in the system allows for more effective disruption of cellular components in the tissue. The change in electric field direction to the cells of the biofilm can maximise breakdown of the cellular components by increasing the area of electropermeabilisation.

It will be appreciated by the person of skill in the art that various modifications may be made to the above-described embodiments without departing from the scope of the present invention. It will be understood by those skilled in the art that the operation of the device and its use have been described with reference to particular values such as voltage, frequency, and power which are provided by way of example only, it will be understood that alternative values may be used. For example, the values may change when the experimental setup is scaled or modified within the scope of the present disclosure. Moreover, it will be understood by those skilled in the art that the absence of moving parts in the exemplary embodiment is indeed by way of example only. In this way it will be understood that the teaching is to be limited only insofar as is deemed necessary in the light of the appended claims.

Similarly the words comprises/comprising when used in the specification are used to specify the presence of stated formations, integers, steps or components but do not preclude the presence or addition of one or more additional formations, integers, steps, components or groups thereof.

It will be understood that while exemplary features of a device for treatment of biofilm have been described that such an arrangement is not to be construed as limiting the invention to such features. The method for treatment of a biofilm may be controlled by a controller implemented in software, firmware, hardware, or a combination thereof. In one mode, the method is implemented in software, as an executable program, and is executed by one or more special or general-purpose digital computer(s), such as a personal computer (PC; IBM-compatible, Apple-compatible, or otherwise), personal digital assistant, workstation, minicomputer, or mainframe computer. The controller may be implemented by a server or computer in which the software modules reside or partially reside.

Generally, in terms of hardware architecture, such a computer will include, as will be well understood by the person skilled in the art, a processor, memory, and one or more input and/or output (I/O) devices (or peripherals) that are communicatively coupled via a local interface. The local interface can be, for example, but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the other computer components.

The processor(s) may be programmed to perform the functions of the method for treatment of microbial contaminants such as biofilm, such as but not limited by actuating provision of the conductive solution, and controlling provision of the PEF and the adjustment of PEF parameters. The processor(s) is a hardware device for executing software, particularly software stored in memory. Processor(s) can be any custom made or commercially available processor, a primary processing unit (CPU), an auxiliary processor among several processors associated with a computer, a semiconductor based microprocessor (in the form of a microchip or chip set), a macro-processor, or generally any device for executing software instructions.

Memory is associated with processor(s) and can include any one or a combination of volatile memory elements (e.g., random access memory (RAM, such as DRAM, SRAM, SDRAM, etc.)) and non-volatile memory elements (e.g., ROM, hard drive, tape, CDROM, etc.). Moreover, memory may incorporate electronic, magnetic, optical, and/or other types of storage media. Memory can have a distributed architecture where various components are situated remote from one another, but are still accessed by processor(s).

The software in memory may include one or more separate programs. The separate programs comprise ordered listings of executable instructions for implementing logical functions in order to implement the functions of the modules. In the example of heretofore described, the software in memory includes the one or more components of the method and is executable on a suitable operating system (O/S).

The present disclosure may include components provided as a source program, executable program (object code), script, or any other entity comprising a set of instructions to be performed. When a source program, the program needs to be translated via a compiler, assembler, interpreter, or the like, which may or may not be included within the memory, so as to operate properly in connection with the O/S. Furthermore, a methodology implemented according to the teaching may be expressed as (a) an object oriented programming language, which has classes of data and methods, or (b) a procedural programming language, which has routines, subroutines, and/or functions, for example but not limited to, C, C++, Pascal, Basic, Fortran, Cobol, Perl, Java, and Ada.

When the method is implemented in software, it should be noted that such software can be stored on any computer readable medium for use by or in connection with any computer related system or method. In the context of this teaching, a computer readable medium is an electronic, magnetic, optical, or other physical device or means that can contain or store a computer program for use by or in connection with a computer related system or method. Such an arrangement can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this disclosure, a "computer-readable medium" can be any means that can store, communicate, propagate, or transport the program for use by or in connection with the instruction execution system, apparatus, or device. The computer readable medium can be for example, but not limited to, an electronic, magnetic, optical, electromagnetic, infrared, or semiconductor system, apparatus, device, or propagation medium. Any process descriptions or blocks in the Figures, should be understood as representing modules, segments, or portions of code which include one or more executable instructions for implementing specific logical functions or steps in the process, as would be understood by those having ordinary skill in the art.

The above detailed description of embodiments of the disclosure is not intended to be exhaustive nor to limit the disclosure to the exact form disclosed. While specific examples for the disclosure are described above for illustrative purposes, those skilled in the relevant art will recognize various modifications are possible within the scope of the disclosure. For example, while processes and blocks have been demonstrated in a particular order, different implementations may perform routines or employ systems having blocks, in an alternate order, and some processes or blocks may be deleted, supplemented, added, moved, separated, combined, and/or modified to provide different combinations or sub-combinations. Each of these processes or blocks may be implemented in a variety of alternate ways. Also, while processes or blocks are at times shown as being performed in sequence, these processes or blocks may instead be performed or implemented in parallel or may be performed at different times. The results of processes or blocks may be also held in a non-persistent store as a method of increasing throughput and reducing processing requirements.

## Claims

1. A device for removing microbial contaminants, comprising:
a non-invasive distal portion including a plurality of electrodes configured to provide a pulsed electric field, PEF, signal on a tissue surface in the vicinity of a microbial contaminant, wherein the PEF penetrates beneath the tissue surface.

2. The device of claim 1, wherein the tissue engaging surfaces are axially moveable relative to a horizontal plane and/or relative to a vertical plane.

3. The device of claim 1 or 2, further comprising an insulating material at least partially surrounding a perimeter of at least one electrode for directing the PEF beneath the surface of the tissue; and/or wherein the electrodes comprise a coating which provides resistance to the adhesion of matter.

4. The device of claim 1 or 2, wherein the electrodes are embedded in an insulating material for directing the PEF beneath the surface of the tissue; and/or wherein the electrodes comprise a coating which provides resistance to the adhesion of matter.

5. The device of claims 1-4, further comprising at least one impedance sensor to assist in determination of PEF parameters to deliver desired current delivery.

6. The device of claim 5, further comprising a controller being configured to change one or more parameters of the PEF signal in response to the at least one impedance sensor detecting an impedance value or a change in impedance.

7. The device of claim 5, the controller being configured to determine electrical properties of tissue based on an impedance value detected by the at least one impedance sensor, and/or the controller being configured to change one or more parameters of the PEF signal in response to determining electrical properties of tissue.

8. The device of claims 1-7, further comprising suction means for providing suction in the vicinity of the microbial contaminants.

9. The device of claims 1-8, the distal portion further comprising:
a. a volume configured for providing an at least partially sealed environment when in abutment with a surface, or
b. a volume configured for providing an at least partially sealed environment when in abutment with a surface, wherein the volume is at least partially formed of a resilient material.

10. The device of claims 1-9, wherein the tissue engaging surfaces are protruding and/or flat relative to the device head and/or comprise a smooth or irregular surface.

11. The device of claims 1-10, further comprising a dispensing means configured to dispense a fluid to the vicinity of the microbial contaminants; and optionally wherein the fluid is a conductive fluid, and/or an antiseptic fluid, such as a saline fluid.

12. The device of claims 1-11 wherein the distal portion of the device head is at least partially formed of an adaptable/flexible material to conform to irregular topographies.

13. The device of claims 1-12 wherein the distal portion of the device contains sensors to detect the physical environment including combinations of temperature, pressure, pH, position of electrodes.

14. The device of any previous claim, further comprising a proximal portion on which the non-invasive distal portion is mounted.

15. The device of any previous claim, further comprising a pump to receive a conductive medium at a variable rate; wherein optionally a motor/battery is connected to the pump for control of the flow rate, wherein when the device comprises a proximal portion optionally the motor/battery is separate from the proximal portion of the device.
